# EUROPEAN PATENT APPLICATION

(11) **EP 4 528 743 A1**
(43) Date of publication of application: **26.03.2025**
(21) Application number: 24201628.5
(22) Date of filing: 20.09.2024
(51) Int. Cl.: G16B 50/00, G16B 30/00

(54) **SEQUENCING METHOD**

(30) Priority: 20.09.2023 CN 202311224731; 15.12.2023 CN 202311732707
(71) Applicant: GeneMind Biosciences Company Limited, Shenzhen City, Guangdong 518023 (CN)
(72) Inventor: Wang, Haijian, Shenzhen City, 518023 (CN); Li, Linsen, Shenzhen City, 518023 (CN); Xu, Jianfeng, Shenzhen City, 518023 (CN); Li, Chengwei, Shenzhen City, 518023 (CN); Wan, Xinchun, Shenzhen City, 518023 (CN); Cai, Jinsen, Shenzhen City, 518023 (CN); Sun, Ruitao, Shenzhen City, 518023 (CN); Li, Yongzhi, Shenzhen City, 518023 (CN)
(74) Representative: Schiweck Weinzierl Koch Patentanwälte Partnerschaft mbB

(57) **Abstract**

The present application relates to the technical field of information and relates to a sequencing method. The sequencing method provided by the present application includes the following steps: using a sequencing system to perform base extension reactions on nucleic acid samples; and outputting sequencing data to an external server at nodes according to preset numbers of base extension cycles, and outputting base calling results by the external server on the basis of the sequencing data. The sequencing method provided by the present application enables the transmission of sequencing intermediate files of the preset numbers of base extension cycles and, by means of the external server, the outputting of the base calling results at nodes and thus the flexible extraction of base calling data for the sequenced samples according to preset requirements, helping increase the efficiency of sequencing of nucleic acid samples.

## Description

### TECHNICAL FIELD

The present disclosure belongs to the technical field of biological information and particularly relates to a sequencing method.

### BACKGROUND

In the related art, automated sequencing platforms determine nucleic acid or protein sequences based on the vast amount of signals or data generated during the sequencing run to obtain sequencing results. Typically, after the sequencing run is completed, the intermediate or result data from this sequencing run are transmitted to another computing device, such as a cloud server, for further processing and analysis. With the advancement of high-throughput sequencing technology, the amount of sequencing data generated in a single sequencing run can be immense, reaching tens of gigabytes (G) or terabytes (T) or even higher magnitudes. Given the current computational transmission technologies, it generally takes much time, even more than 20 hours, to transmit sequencing intermediate or result data from the sequencing platform to another computing device. Therefore, the efficient transmission of large volumes of data, such as sequencing data, is now a concern.

### SUMMARY

The present application provides a method for transmitting sequencing data, aiming to address the technical problem that after completing the sequencing process, existing sequencers need to transmit the sequencing data to cloud servers, which results in very long waiting times for data transmission and an increased risk of data loss.

To achieve the purpose of the present application described above, the embodiments of the present application use the following technical solutions:
A sequencing method, including the following steps:
using a sequencing system to perform base extension reactions on nucleic acid samples; and
outputting sequencing data to an external server at nodes according to preset numbers of base extension cycles, and outputting base calling results by the external server on the basis of the sequencing data.

The sequencing method provided by the present application enables efficient and prompt transmission of sequencing data and avoids the situation where it takes a long time to wait for the sequencing run to be completed, by transmission of the sequencing data generated at each node to the external server, in comparison with a method of waiting for the sequencing run to finish before transmitting the sequencing data. Therefore, the method provided by the present application supports simultaneous sequencing and transmission of data to a specified server, supports the server's processing of the sequencing data to output the base calling results, helps accelerate the realization of the entire sequencing detection process, and can effectively avoid significant data loss.

### BRIEF DESCRIPTION OF THE DRAWING

To more clearly illustrate the technical solutions in the embodiments of the present application, the drawing required for use in the description of the embodiments or the prior art will be briefly described below. It is obvious that the drawing in the description below is only some embodiments of the present application, and other drawings can be derived from the drawing by those of ordinary skill in the art without creative efforts.

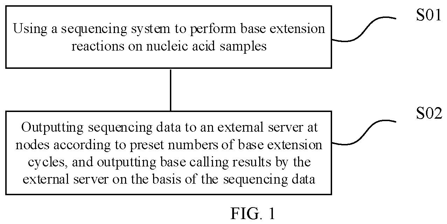

FIG. 1 is a schematic flow chart of a sequencing method provided in an embodiment of the present application.

### DETAILED DESCRIPTION

In order to make the technical problems, technical solutions, and beneficial effects to be solved by the present application more apparent, the present application will be further described in detail below with reference to the embodiments. It should be understood that the specific embodiments described herein are intended to explain the present application only rather than limit the present application.

In the present application, the terms used in the embodiments of the present application are for the purpose of describing particular embodiments only and are not intended to be limiting to the present application. The term "and/or" describes an associative relationship between associated objects, and means that there may be three relationships, for example, A and/or B may represent that: A is present alone, A and B are present simultaneously, and B is present alone. A and B can be singular or plural. The character "/" generally indicates a "and" relationship between the associated objects. The term "at least one" means one or more, and "a plurality of" means two or more. "At least one" or similar expressions thereof refer to any combination of these items, including any combination of the singular or plural items. For example, "at least one of a, b, or c" or "at least one of a, b, and c" may each refer to: a, b, c, a-b (i.e., a and b), a-c, b-c, or a-b-c, where a, b, and c may be a single item or a plurality of items, respectively.

As used in the embodiments and the appended claims of the present application, the singular forms "a", "an", and "the" are intended to include the plural forms as well, unless the context clearly indicates otherwise.

The terms "first" and "second" are used for description purposes only, are used for distinguishing purposes such as substance, orientation, interface, message, request, and terminal from one another, and should not be construed as indicating or implying relative importance or implicitly indicating the number of technical features indicated. For example, a first nucleic acid sample may be referred to as a second nucleic acid sample, and a second nucleic acid sample may be referred to as a first nucleic acid sample, without departing from the scope of the embodiments of the present application. Therefore, features defined with "first" and "second" may explicitly or implicitly include one or more of the features.

It should be understood that in various embodiments of the present application, the sequence numbers of the processes described above do not imply an execution sequence, some or all of the steps may be executed in parallel or executed sequentially, and the execution sequence of each process should be determined by its function and inherent logic and should not constitute any limitation to the implementation process of the embodiments of the present application.

In the embodiments of the present application, the term "sequencing" may also be referred to as "nucleic acid sequencing" or "gene sequencing". The three are used interchangeably and refer to the determination of the type and order of bases or nucleotides (including nucleotide analogs) in a nucleic acid molecule. The sequencing involves the process of binding nucleotides to a nucleic acid template and acquiring the corresponding signals emitted by the nucleotides (including analogs). The sequencing includes sequencing by synthesis (SBS) and/or sequencing by ligation (SBL); includes DNA sequencing and/or RNA sequencing; includes long fragment sequencing and/or short fragment sequencing (the long fragment and short fragment are defined relatively; for example, nucleic acid molecules longer than 1 Kb, 2 Kb, 5 Kb or 10 Kb may be referred to as long fragments, and nucleic acid molecules shorter than 1 Kb or 800 bp may be referred to as short fragments); and includes double-end sequencing, single-end sequencing, paired-end sequencing, and/or the like, where the double-end sequencing or paired-end sequencing may refer to the reading of any two segments or portions of the same nucleic acid molecule that are not completely overlapping.

The sequencing generally involves cycles of process to achieve the determination of the type and order of multiple bases or nucleotides on a nucleic acid template. The embodiments of the present application refer to each cycle of the "process to achieve the determination of the type and order of multiple bases or nucleotides on a nucleic acid template" as "one cycle of sequencing". "One cycle of sequencing", also referred to as "sequencing cycle" or "one base extension cycle", may be defined as one base extension of four types of nucleotides/bases; in other words, "one cycle of sequencing" may be defined as the determination of the base or nucleotide type at any given position on the template. For sequencing platforms that achieve sequencing based on polymerization or ligation reactions, the cycle of sequencing includes the process of binding four types of nucleotides (including nucleotide analogs) to the nucleic acid template at a time through base complementation and acquiring the corresponding signals emitted. For platforms that achieve sequencing based on polymerization reaction, a reaction system includes reaction substrate nucleotide, polymerase, and a nucleic acid template. A sequence fragment (a sequencing primer) binds to the nucleic acid template, and based on the base pairing rules and the principle of polymerization reaction, the added reaction substrate nucleotides are connected to the sequencing primer under the catalysis of the polymerase to realize the binding of a nucleotide to a specific position on the nucleic acid template. Generally, one cycle of sequencing may include one or more base extensions (repeats). For example, four types of nucleotides are sequentially added to the reaction system to each perform base extensions and corresponding acquisition of reaction signals, and one cycle of sequencing includes four base extensions; for another example, four types of nucleotides are added into the reaction system in any combinations (such as in pairs or in one-three combinations), the two combinations each perform base extensions and corresponding acquisition of reaction signals, and one cycle of sequencing includes two base extensions; for yet another example, four types of nucleotides are added simultaneously to the reaction system for base extension and reaction signal acquisition, and one cycle of sequencing includes one base extension. The term "nucleic acid sample" means a polynucleotide to be sequenced, which is part or all of a nucleic acid molecule. Polynucleotides generally consist of a specific sequence of four types of nucleotide bases: adenine (A); cytosine (C); guanine (G); and thymine (T) (when the polynucleotide is RNA, uracil (U) is correspondingly changed to thymine (T)). Thus, a polynucleotide sequence is an alphabetical representation of a polynucleotide molecule; alternatively, the term may apply to the polynucleotide molecule itself. This alphabetical representation can be input into databases in a computer having a central processing unit and used for bioinformatics applications, such as functional genomics and homology searching. Polynucleotides may optionally include one or more non-standard nucleotides, nucleotide analogs, and/or modified nucleotides.

The term "nucleic acid molecule" refers to a nucleotide polymer of any length, which may include ribonucleotides or analogs thereof, deoxyribonucleotides or analogs thereof, and mixtures of the foregoing nucleotides or analogs thereof. Nucleotides in a nucleic acid molecule may include naturally occurring nucleotides and functionally alternative analogs thereof. Examples of analogs can hybridize to nucleic acids in a sequence-specific manner, or can be used as templates for the replication of particular nucleotide sequences. Naturally occurring nucleotides generally have a backbone containing a phosphodiester bond. Analog structures may have alternative backbone linkages including any types known in the art. Naturally occurring nucleotides generally have deoxyribose (e.g., found in DNA) or ribose (e.g., found in RNA). Analog structures may have alternative sugar moieties including any types known in the art. Nucleotides may contain natural bases or non-natural bases. Bases in natural DNA may include one or more of adenine, thymine, cytosine, and/or guanine, and bases in natural RNA may include one or more of adenine, uracil, cytosine, and/or guanine. Any non-natural base or base analog may also be used for a nucleotide, such as a locked nucleic acid (LNA) and a bridged nucleic acid (BNA).

The term "nucleic acid template" refers to a master nucleic acid molecule or master nucleic acid molecule fragment that binds to nucleotides or nucleotide analogs through a plurality of successive base extension cycles. The nucleic acid template may be the entire sequence of the nucleic acid molecule or a partial fragment of the nucleic acid molecule. The "nucleic acid template" may be a nucleic acid fragment used as a template in extension reactions in which sequencing by synthesis is performed; since the base added to the extension reactions and the sequencing template satisfy the base pairing rules, the sequence of the sequencing template can be determined by determining the type of the base added to each extension cycle. The sequence of the nucleic acid template herein may generally include, but is not limited to, at least one of the sequence of the target molecule, the UMI sequence, and the sample tag sequence, sometimes also referred to herein as an "insert" or "target molecule".

The term "base", also known as "nucleobase", refers to a purine or pyrimidine compound or a derivative thereof, which is a constituent of nucleic acid (i.e. DNA or RNA or a derivative thereof). In an embodiment of the present application, the base may be a naturally occurring DNA or RNA base or a derivative of a naturally occurring DNA or RNA base (e.g., a base analog). In some embodiments, the base is capable of forming at least one hydrogen bond with a complementary base (e.g., adenine hydrogen bonds with thymine, adenine hydrogen bonds with uracil, or guanine pairs with cytosine). Non-limiting examples of the base include moieties of cytosine or a derivative thereof (e.g., a cytosine analog), guanine or a derivative thereof (e.g., a guanine analog), adenine or a derivative thereof (e.g., an adenine analog), thymine or a derivative thereof (e.g., a thymine analog), uracil or a derivative thereof (e.g., a uracil analog), hypoxanthine or a derivative thereof (e.g., a hypoxanthine analog), xanthine or a derivative thereof (e.g., a xanthine analog), guanosine or a derivative thereof (e.g., a 7-methylguanosine analog), deaza-adenine or a derivative thereof (e.g., a deaza-adenine analog), deaza-guanine or a derivative thereof (e.g., deaza-guanine), deaza-hypoxanthine or a derivative thereof, 5,6-dihydrouracil or a derivative thereof (e.g., a 5,6-dihydrouracil analog), 5-methylcytosine or a derivative thereof (e.g., a 5-methylcytosine analog), or 5-hydroxymethylcytosine or a derivative thereof (e.g., a 5-hydroxymethylcytosine analog). In embodiments, the base is thymine, cytosine, uracil, adenine, guanine, hypoxanthine, xanthine, theobromine, caffeine, uric acid, or isoguanine.

The term "primer", also known as "probe", refers to an oligonucleotide or a nucleic acid molecule that can hybridize to a target sequence of interest. In an embodiment, the primer functions as a substrate onto which nucleotides may be polymerized by a polymerase. For example, the primer may be used as a starting point for DNA or RNA synthesis. For example, a sequencing primer can hybridize to the synthesized nucleic acid template strand so as to trigger the synthesis of a new strand complementary to the synthesized nucleic acid template strand. The primer may include any combination of nucleotides or analogs thereof. In some examples, the primer is a single-stranded oligonucleotide or polynucleotide.

The term "file" is equivalent to data, including any information that can be stored, transmitted, read, or processed, including static data and dynamic data, including file data and program data, including any type of file, such as web pages, pictures, text, audio, mail, compressed data, libraries, numbers, letters, analog quantities, algorithms, instructions, programs, and the like.

Referring to FIG. 1, the embodiments of the present application provide a sequencing method, including:
S01: using a sequencing system to perform base extension reactions on nucleic acid samples.

In an embodiment of the present application, the sequencing system refers to a system for running a sequence determination program for nucleic acid samples, which is generally represented by an apparatus into which the system is built. For example, the sequencing system may be referred to as a sequencing platform or understood as a sequencing apparatus or sequencer.

The sequencing platform for sequencing in the embodiments of the present application is not specifically limited, and theoretically, the method can be applied to any sequencing platform for nucleic acid sequencing based on the principle that the complementary pairing of nucleotide sequences is performed based on base extension reactions.. According to the embodiments of the present application, available sequencing platforms include, but are not limited to, the Hiseq, Miseq, Nextseq, and Novaseq sequencing platforms of Illumina, the Ion Torrent platform of Thermo Fisher/Life Technologies, the BGISEQ and MGISEQ/DNBSEQ platforms of BGI, and single-molecule sequencing platforms.

In an embodiment of the present application, using a sequencing system to perform base extension reactions on nucleic acid samples refers to using the sequencing system to run a program as follows: mixing a nucleic acid template complex, where a sequencing primer is bound to a nucleic acid template, with substrate nucleotides and a polymerase, and allowing the added substrate nucleotides to sequentially extend on the sequencing primer under the catalysis of the polymerase, based on the base pairing rules and the principle of polymerization reaction, to achieve the binding of the nucleotides to the specific positions of the nucleic acid template. In some embodiments, the nucleic acid template is provided with a primer-binding site, and the sequencing system-run program further includes: binding the sequencing primer to the primer-binding site to form the nucleic acid template complex.

As the conversion of a sequencing intermediate file into a final file needs to be performed synchronously during sequencing, when nucleic acid samples with different numbers of bases or different sequence lengths are sequenced, it is usually necessary to wait for the sequencing of the nucleic acid sample with the greatest number of bases or the longest sequence to finish before obtaining the final base calling result. This undoubtedly increases the length of time taken for the sequencing of nucleic acid samples with smaller numbers of bases, reducing the sequencing efficiency in multi-sample detection. In view of this, the embodiments of the present application provide a sequencing method for a scenario where a sequencing system is used to perform base extension reactions on multiple nucleic acid samples. The method enables the transmission of sequencing intermediate files of preset numbers of base extension cycles and, by means of an external server, the outputting of base calling results at nodes and thus the flexible extraction of base calling data for the sequenced samples according to preset requirements, helping increase the efficiency of simultaneous sequencing of multiple nucleic acid samples.

For the nucleic acid template, in one embodiment, the nucleic acid template includes a nucleic acid sample to be sequenced; that is, during sequencing, a nucleic acid molecule containing the nucleic acid sample to be sequenced is used as the nucleic acid template, base extension reactions are performed on the nucleic acid template, and the sequence of the nucleic acid sample to be sequenced is determined through base calling results of the base extension reactions. In this embodiment, the sequence of the nucleic acid sample to be sequenced is a sequence complementary to a sequence that is formed by sequentially arranging the read nucleotides involved in the base extension reactions in an order. In another embodiment, the nucleic acid template includes a complementary strand of a nucleic acid sample to be sequenced; that is, during sequencing, a nucleic acid molecule containing the complementary strand of the nucleic acid sample to be sequenced is used as the nucleic acid template, base extension reactions are performed on the nucleic acid template, and the sequence of the nucleic acid sample to be sequenced is determined through base calling results of the base extension reactions. In this embodiment, the sequence of the nucleic acid sample to be sequenced is a sequence of the read nucleotides involved in the base extension reactions. In this embodiment, the sequence of the nucleic acid sample to be sequenced is a sequence that is formed by sequentially arranging the read nucleotides involved in the base extension reactions in an order.

In some possible embodiments, one nucleic acid sample corresponds to one nucleic acid template; that is, one nucleic acid template includes only one nucleic acid sample. When the nucleic acid sample is sequenced, the obtained base calling result is a sequencing result for the nucleic acid sample.

In another possible embodiment, one nucleic acid template contains two or more nucleic acid samples; that is, the nucleic acid molecule used as the nucleic acid template contains the sequences of two or more nucleic acid samples. The base calling result obtained by sequencing the nucleic acid samples includes sequencing results for the two or more nucleic acid samples described above. In this embodiment, the nucleic acid template complex includes at least one sequencing primer bound to the nucleic acid template. As one implementation, one sequencing primer is bound to the nucleic acid template, in which case during base extension reactions, the substrate nucleotides participate in the reactions along the 3' end of the sequencing primer and are extended one by one. As another implementation, two or more sequencing primers are bound to the nucleic acid template, and different nucleic acid samples are separated by the sequencing primers, in which case during base extension reactions, the substrate nucleotides participate in the reactions along the 3' end of each of the sequencing primers and are extended one by one to realize simultaneous sequencing of multiple regions of the nucleic acid template.

The type and source of the nucleic acid samples in the embodiments of the present application are not strictly limited, and the nucleic acid samples may be DNA fragments, or may be RNA fragments, or may contain both DNA fragments and RNA fragments. The nucleic acid samples may be identical or different DNA/RNA fragments of the same species from the same source, or may be identical or different DNA/RNA fragments of different species from the same source, or may even be DNA/RNA samples from unknown sources.

In an embodiment of the present application, each nucleic acid sample includes a sample sequence derived from the sample, and one tag sequence at one end of the nucleic acid sequence, where the sample sequence and the tag sequence are bound, e.g., covalently connected, by adjacent nucleotides. The tag sequence is used for marking sample sequence information, such as sample library assignment of the sample sequence. When a sequencing system is used to perform base extension reactions on a nucleic acid sample, sequencing of the tag sequence is completed first, and therefore when base calling results are output at nodes, information assignment of the nucleic acid sample is determined based on the determined tag sequence. In particular, in the case that the sequencing of some of the nucleic acid samples has been completed and the sequencing of the remaining nucleic acid samples has not yet been completed, information of the nucleic acid samples the sequencing of which has been completed can be assigned based on the tag sequences that have been determined.

Certainly, it should be understood that tag sequences may be arranged at both ends of the sequence of the nucleic acid sample.

In some implementations, a nucleic acid template includes one nucleic acid sample, and the nucleic acid sample contains tag sequences at both ends. Thus, the nucleic acid sample can be double-end sequenced.

In some other implementation, two or more nucleic acid samples are located on the same nucleic acid template, and the sequences of the nucleic acid samples contain tag sequences at both ends.

As one implementation, the two tag sequences at both ends of the sequence of the nucleic acid sample are tag sequences of two nucleic acid samples, where one of the tag sequences is the nucleic acid sample's own tag sequence, and the other is the tag sequence of another nucleic acid sample that is adjacent to the nucleic acid sample. As one embodiment, the nucleic acid template includes a nucleic acid sample 1 and a nucleic acid sample 2 which are connected, where the nucleic acid sample 1 includes a tag sequence 1 at the 3' end, the nucleic acid sample 2 includes a tag sequence 2 at the 3' end, and the 5' end of the nucleic acid sample 1 is connected to the 3' end of the nucleic acid sample 2. When a sequencing system is used to perform base extension reactions on a plurality of nucleic acid samples, the base extension reactions may be synchronously performed on the nucleic acid sample 1 and the nucleic acid sample 2, and base calling reactions of the tag sequence 1 and the tag sequence 2 are completed first.

As another embodiment, the nucleic acid template includes a nucleic acid sample 1 and a nucleic acid sample 2 which are connected, where the nucleic acid sample 1 includes a tag sequence 1 at the 5' end, the nucleic acid sample 2 includes a tag sequence 2 at the 3' end, and the 5' end of the nucleic acid sample 1 is connected to the 3' end of the nucleic acid sample 2. When a sequencing system is used to perform base extension reactions on a plurality of nucleic acid samples, the base extension reactions may be synchronously performed on the nucleic acid sample 1 and the nucleic acid sample 2, and base calling reactions of the tag sequence 1 and the tag sequence 2 are completed first.

By the method described above, two nucleic acid samples on one nucleic acid template can be sequenced simultaneously, and when the two nucleic acid samples have different sequence lengths, base calling results are output at nodes, so that the sequence information of the nucleic acid sample with the shorter sequence length can be obtained first, without the need to wait for the sequencing of the entire nucleic acid template to be completed.

It should be understood that when the nucleic acid template contains three or more nucleic acid samples, sequencing can also be performed by the method described above, and base calling results are output at nodes to asynchronously obtain base calling results for each of the nucleic acid samples, thereby improving the efficiency of base calling.

In one possible embodiment, one end or both ends of the nucleic acid sample sequence contain(s) a known sequence, and the known sequence may also be referred to as an adapter or sequencing adapter. The known sequence, used as a primer-binding site, enables the nucleic acid sample to capture a sequencing primer or causes the nucleic acid sample to be captured by a sequencing primer, so that base extension reactions are performed under the action of a polymerase. In one embodiment of the present application, before using the sequencing system to perform base calling on the nucleic acid sample, the method further includes: treating the nucleic acid sample. The embodiment includes at least the following implementation: the known sequence is located at the 3' end of the nucleic acid sample.

In one possible embodiment, the nucleic acid sample is fixed to a solid carrier, so that the nucleic acid sample is suitable for being loaded to a sequencing platform for sequencing. The nucleic acid sample may be fixed to the solid carrier by chemical linkage, e.g., by covalent binding between a terminal nucleotide of the nucleic acid template where the nucleic acid sample is present and a biomolecule and/or a chemical molecule on the surface of the solid carrier. Illustratively, a terminal nucleotide of the nucleic acid template is bonded to a biomolecule fixed to the surface of the solid carrier. The nucleic acid sample may also be fixed to the solid carrier by hydrogen bonding, electrostatic adsorption, or the like. Illustratively, a terminal nucleotide of the nucleic acid template is hydrogen-bonded to a sequencing primer fixed to the surface of the solid carrier. In an embodiment of the present application, the solid carrier may be understood as a substrate capable of fixing a nucleic acid sample; in some implementations, the solid carrier can also provide a reaction site for base extension reactions of the nucleic acid sample. In some expressions, the solid carrier may also be expressed as a chip, biochip, sequencing chip, or flow cell.

In some embodiments, the solid carrier is provided with a plurality of liquid flow channels, which are sites where base extension reactions are performed for sequencing, and for a plurality of nucleic acid samples, the plurality of nucleic acid samples may be randomly placed in the plurality of liquid flow channels, or the plurality of nucleic acid samples may be distributed in different liquid flow channels. As one implementation, nucleic acid templates derived from different libraries may be arranged in different channels, respectively, or different nucleic acid templates may be fixed into the same channel.

In an embodiment of the present application, when a nucleic acid template containing a nucleic acid sample is fixed to the surface of a solid carrier, the nucleic acid template randomly hybridizes to a primer probe fixed to the surface of the solid carrier and is thus fixed to a certain position, which is referred to as a site. The solid carrier contains a plurality of sites for fixing nucleic acid templates. It should be understood that the number of nucleic acid templates at the sites to which the nucleic acid templates are fixed is not strictly limited. For one implementation, for each site on the solid carrier, the number of nucleic acid samples may be one. In this case, when base extension reactions are performed, the base extension reactions are performed at the site on a nucleic acid template where the nucleic acid sample is present, and one complementary strand is generated. However, this does not mean that the number of nucleic acid templates containing the nucleic acid sample on the solid carrier is one. For another implementation, for each site on the solid carrier, the number of nucleic acid samples may be more than one. In this case, when base extension reactions are performed, the base extension reactions are simultaneously performed at the site on nucleic acid templates where the plurality of nucleic acid samples are present, and a plurality of complementary strands corresponding to the number of the nucleic acid templates are generated.

A base extension reaction can be understood as a process of using the 3' end of a sequencing primer bound to a nucleic acid template as a sequencing origin and extending a nucleic acid strand at the 3' end of the sequencing primer under the action of a polymerase based on the base complementary pairing rules to synthesize a complementary strand of the nucleic acid template. In an embodiment of the present application, the method for base extension reactions is not strictly limited, and the current base extension methods for generating complementary strands of nucleic acid templates based on the base complementation rules can all be used in the embodiments of the present application. For example, in the embodiments of the present application, base extension reactions may be performed based on polymerization reactions or ligation reactions. In one cycle of sequencing, the four types of substrate nucleotides (G, C, A, and T/U) may be added in one step for base extension reactions to complete one cycle of sequencing; or the four types of substrate nucleotides may be combined in pairs and added in two steps for base extension reactions to complete one cycle of sequencing, with two types of substrate nucleotides added in each step, where the way to combine the four types of substrate nucleotides in pairs is not strictly limited; or the four types of substrate nucleotides may be subjected to one-three combination, which means that one type of substrate nucleotides is assigned to the first combination and the remaining three types of substrate nucleotides are assigned to the second combination, in which case the first combination (or the second combination) of substrate nucleotides is added in the first step for base extension reactions, and the second combination (or the first combination) of substrate nucleotides is added in the second step for base extension reactions, so that the four types of substrate nucleotides are added in two steps to complete one cycle of sequencing, where the way to perform the one-three combination of the four types of substrate nucleotides is not strictly limited; or the four types of substrate nucleotides may be added in four steps, respectively, for base extension reactions.

In the embodiments of the present application, using a sequencing system to perform base extension reactions on a plurality of nucleic acid samples may be divided into a plurality of cases according to the number of sequencing systems.

As one implementation, one sequencing system is used to perform base extension reactions on a plurality of nucleic acid samples simultaneously; that is, the base extension reactions are performed on the plurality of nucleic acid samples on one sequencer, and base calling results of these nucleic acid samples are output in the following step. In this case, the plurality of nucleic acid samples undergo base extension synchronously; that is, the cycle number of each cycle of sequencing of different nucleic acid samples is synchronous. Illustratively, a first sequencing system is used to start base extension reactions of S nucleic acid samples simultaneously, and the numbers of sequencing cycles for the S nucleic acid samples are synchronous.

As another implementation, two or more sequencing systems are used to perform base extension reactions on a plurality of nucleic acid samples. The nucleic acid samples are divided into two groups, namely a first group of nucleic acid samples and a second group of nucleic acid samples. For example, two sequencing systems (a first sequencing system and a second sequencing system) are used to sequence the nucleic acid samples: the first sequencing system is used to perform base extension reactions on the first group of nucleic acid samples, in which case the first group of nucleic acid samples undergo base extension synchronously; that is, the number of sequencing cycles for each nucleic acid sample in the first group of nucleic acid samples is synchronous. The second sequencing system is used to perform base extension reactions on the second group of nucleic acid samples, in which case the second group of nucleic acid samples undergo base extension synchronously; that is, the number of sequencing cycles for each nucleic acid sample in the second group of nucleic acid samples is synchronous. However, whether the number of sequencing cycles for the first group of nucleic acid samples synchronizes with that for the second group of nucleic acid samples depends on the times for the first sequencing system and the second sequencing system to start the base extension reactions on the first group of nucleic acid samples and the second group of nucleic acid samples, respectively, and the setting of the sequencing systems with which base extension reactions are performed on the nucleic acid samples. Thus, there is at least the case that the number of sequencing cycles for the first group of nucleic acid samples does not synchronize with the number of sequencing cycles for the second group of nucleic acid samples. Certainly, it should be understood that when the number of sequencing systems is more than two, these sequencing systems can also be adapted for the case described above. The number of sequencing systems can be flexibly adjusted based on the actual application requirements, and the number of sequencing systems is not limited in the embodiments of the present application.

In an embodiment of the present application, for at least some of the nucleic acid samples, base extension reactions are performed through the same sequencing system; that is, the base extension reactions are started simultaneously for at least some of the nucleic acid samples, and the numbers of sequencing cycles for these nucleic acid samples are synchronous.

S02: outputting sequencing data to an external server at nodes according to preset numbers of base extension cycles, and outputting base calling results by the external server on the basis of the sequencing data.

Unlike the manner in which base calling results are output together after base extension reactions of all nucleic acid samples are finished, in the embodiments of the present application, when preset numbers of base extension cycles have been completed for some of the nucleic acid samples or some sequences of the nucleic acid samples, base calling results of those the base extension reactions of which have been completed are output.

In an embodiment of the present application, a plurality of nucleic acid samples are provided, and the preset numbers of base extension cycles are numbers of base extension cycles required for predetermined outputs of the nucleic acid samples. The preset number of base extension cycles may be set according to the characteristics of the source of the sample or set based on the sequence length of the nucleic acid sample, as well as the function or purpose of the information that needs to be obtained, and the embodiments of the present application do not impose strict limitations on this. As one implementation, when the same sequencing chip contains nucleic acid samples derived from different species or the needed sequencing information has different applications, based on the difference in detection type, the mere partial sequencing of some of the nucleic acid samples yields the needed sequencing information; therefore, different preset numbers of base extension cycles may be set according to the sequencing requirements. Illustratively, when the sequencing chip contains one or more of a plurality of viral nucleic acid samples, human gene samples, plant gene samples, and the like, based on different uses of the needed sequencing information, the mere completion of sequencing of part of the sequences of some of the nucleic acid samples allows the sequencing of those samples to be finished, in which case different preset numbers of base extension cycles may be set based on the sequence lengths required for the sequencing of these different nucleic acid samples.

In some embodiments, at least one preset number of base extension cycles is set for each nucleic acid sample. In one embodiment, a plurality of preset numbers of base extension cycles may be set for a nucleic acid sample, and when base calling results are output at nodes, base calling results are output once each time one preset number of base extension cycles is reached. It should be understood that the output base calling results are base calling results between the previous preset number of base extension cycles and the current preset number of base extension cycles, and include base calling results of sequencing cycles corresponding to the current preset number of base extension cycles. When base calling results are the first output, the output base calling results include base calling results from the start of the base calling reactions to the point when the preset number of base extension cycles is reached. In another embodiment, one preset number of base extension cycles is set for each nucleic acid sample; that is, for each nucleic acid sample, a preset number of sequencing cycles required to obtain base calling results is determined according to the expected needed sequencing information.

In an embodiment of the present application, at least some of the plurality of nucleic acid samples on which a sequencing system is used to perform base extension reactions have different preset numbers of base extension cycles, and in this way, outputting base calling results at nodes enables part or all of the sequence information of the nucleic acid samples to be output at different time nodes and earlier application of the output sequencing data, without waiting for the sequencing of all the nucleic acid samples to be completed, thereby improving the overall sequencing efficiency. In a first possible embodiment, base extension reactions are started simultaneously for at least some of the nucleic acid samples. It should be understood that each nucleic acid sample includes one tag sequence at one end, and the sequencing of the tag sequence is completed earlier than the sequencing of a sample sequence of the nucleic acid sample, so that base calling results of nucleic acid samples for which the preset numbers of base extension cycles have been achieved can be assigned or subjected to other analyses based on the tag sequences.

In this case, outputting sequencing data to an external server at nodes according to preset numbers of base extension cycles, and outputting base calling results by the external server on the basis of the sequencing data include:
in the order in which the preset numbers of base extension cycles occur, when the preset number of base extension cycles are completed for each of the nucleic acid samples, outputting sequencing data of a current node to the external server, and outputting the base calling results by the external server on the basis of the sequencing data, with base extension reactions of the nucleic acid samples the sequencing of which has not been completed being not stopped.

The method can be divided into several cases according to the preset numbers of base extension cycles set for the nucleic acid samples.

In one implementation, one preset number of base extension cycles is set for each of the nucleic acid samples for which base extension reactions are started simultaneously, and at least some of the nucleic acid samples have different preset numbers of base extension cycles. In this case, in ascending order according to the preset numbers of base extension cycles, base calling results for a certain number of base extension cycles are sequentially output. Illustratively, base extension reactions are started simultaneously for five nucleic acid samples, and for the five nucleic acid samples, different preset numbers of base extension cycles C1, C2, C3, C4, and C5 are set, respectively, and satisfy C1 < C2 < C3 < C4 < C5. In this case, when the number of base extension cycles is C1, base calling results of the first cycle to cycle C1 are output, and at the same time, the base extension reactions of the nucleic acid samples the sequencing of which has not been completed are not stopped; when the number of base extension cycles reaches C2, base calling results of cycle C1 to cycle C2 are output, and at the same time, the base extension reactions of the nucleic acid samples the sequencing of which has not been completed are not stopped; when the number of base extension cycles reaches C3, base calling results of cycle C2 to cycle C3 are output, and at the same time, the base extension reactions of the nucleic acid samples the sequencing of which has not been completed are not stopped; when the number of base extension cycles reaches C4, base calling results of cycle C3 to cycle C4 are output, and at the same time, the base extension reactions of the nucleic acid samples the sequencing of which has not been completed are not stopped; when the number of base extension cycles reaches C5, base calling results of cycle C4 to cycle C5 are output, and at the same time, the base extension reactions of the nucleic acid samples the sequencing of which has not been completed are not stopped. It should be understood that for a nucleic acid sample for which a preset number of base extension cycles has not been reached, corresponding base calling results may be output when preset numbers of base extension cycles of other nucleic acid samples are achieved; for example, for a nucleic acid sample with a preset number of base extension cycles of C3, base calling results may be output at three nodes: C1, C2, and C3; alternatively, base calling results may not be output when preset numbers of base extension cycles of other samples are achieved, and instead, all base calling samples are output together when the nucleic acid sample's own preset number of base extension cycles is achieved; for example, for a nucleic acid sample with a preset number of base extension cycles of C3, base calling results are not output at the two nodes C1 and C2, but only when the number of base extension cycles reaches C3, base calling results of the nucleic acid sample are output in one step.

In another implementation, two or more preset numbers of base extension cycles are set for at least some of the nucleic acid samples for which base extension reactions are started simultaneously. In this case, similarly, in ascending order according to the preset numbers of base extension cycles of the nucleic acid samples for which base extension reactions are started simultaneously, base calling results for a certain number of base extension cycles are sequentially output. Illustratively, base extension reactions are started simultaneously for three nucleic acid samples; one preset number of base extension cycles, C1, is set for the first nucleic acid sample, two preset numbers of base extension cycles, C2 and C3, are set for the second nucleic acid sample, and three preset numbers of base extension cycles, C4, C5, and C6, are set for the third nucleic acid sample. When C1, C2, C3, C4, C5, and C6 satisfy C1 < C2 < C3 < C4 < C5 < C6, base calling results are output when each of the preset numbers of base extension cycles is achieved. When some of the preset numbers of base extension cycles of different nucleic acid samples overlap, for example, C2 = C4, base calling results for a certain number of base extension cycles are sequentially output in ascending order according to C1, C2 (C4), C3, C5, and C6. The base calling results for a certain number of base extension cycles refer to base calling results corresponding to the base extension reactions from the first base extension cycle started after the previous preset number of base extension cycles ends to the preset number of base extension cycles. When the current preset number of base extension cycles is the minimum value, for example, C1, base calling results of the base extension reactions from the first base extension cycle to the preset number of base extension cycles, for example, from the first cycle to cycle C1, are output. Similarly, for a nucleic acid sample for which a preset number of base extension cycles has not been reached, corresponding base calling results may be output when preset numbers of base extension cycles of other nucleic acid samples are achieved; for example, for a nucleic acid sample with preset numbers of base extension cycles of C2 and C3, base calling results may be output at C1; alternatively, base calling results may not be output when preset numbers of base extension cycles of other samples are achieved, and instead, all base calling samples are output when the nucleic acid sample's own preset numbers of base extension cycles are achieved; for example, for a nucleic acid sample with preset numbers of base extension cycles of C2 and C3, base calling results are not output when the number of base extension cycles reaches C1, but when the number of base extension cycles reaches C2 or C3, base calling results of the nucleic acid sample are sequentially output.

In an embodiment of the present application, the preset numbers of base extension cycles of the nucleic acid sample may be set in advance. For a nucleic acid sample containing a plurality of preset numbers of base extension cycles, the first preset number of base extension cycles may be set in advance, or set based on the preset numbers of base extension cycles of other nucleic acid samples that occur earlier than the first preset number of base extension cycles; the second preset number of base extension cycles or later preset number(s) of base extension cycles may be all set in advance, or set based on the base calling results corresponding to the first preset number of base extension cycles. Illustratively, when a nucleic acid sample includes a first preset number of base extension cycles, and when base calling result data corresponding to the first preset number of base extension cycles are insufficient to achieve its application purpose or its application effect, a second preset number of base extension cycles may be set on the basis of the first preset number of base extension cycles to collect the base calling results of the sequencing cycles between the first preset number of base extension cycles and the second preset number of base extension cycles for further analysis until its application purpose or the desired effect is achieved.

For some embodiments, some nucleic acid samples have a preset number of base extension cycles that is equal to the number of bases of the nucleic acid samples. The number of bases of the nucleic acid samples refers to the number of bases constituting the nucleic acid samples, may also refer to the number of nucleotides forming the nucleic acid molecules, and may be characterized by the sequence length of the nucleic acid molecules. In this case, outputting base calling results for the preset numbers of base extension cycles of these nucleic acid samples enables the base calling results of these nucleic acid samples to be output in advance without interference before the sequencing of other nucleic acid samples has not been completed, thereby improving the overall sequencing efficiency. In this case, similarly, in ascending order according to the preset numbers of base extension cycles of the nucleic acid samples, base calling results of the current node are output when the preset number of base extension cycles are completed for each of the nucleic acid samples.

For a nucleic acid sample with a preset number of base extension cycles that is equal to the number of bases of the nucleic acid sample, particularly when the preset number of base extension cycles of each of the nucleic acid samples is equal to the number of bases of the nucleic acid sample, outputting sequencing data to an external server at nodes according to preset numbers of base extension cycles, and outputting base calling results by the external server on the basis of the sequencing data include:
in ascending order according to the numbers of bases, sequentially completing sequencing of the nucleic acid samples of various numbers of bases, and in the final base extension cycle of the nucleic acid sample corresponding to each of the numbers of bases and when the base extension reactions of the nucleic acid samples the sequencing of which has not been completed are ongoing,
outputting the sequencing data to the external server; and outputting the base calling results by the external server on the basis of the sequencing data.

It should be understood that the completion of sequencing referred to in the embodiments of the present application refers to obtaining base calling results required for preset results. When the base calling results required for the preset results have been obtained, the sequencing can still be considered to be completed even if the determination of all the nucleotide types in the sequence of the nucleic acid sample has not been completed.

It should be understood that the base calling output results of each node may be output after the current preset number of base extension cycles is achieved and before the next preset number of base extension cycles is achieved.

In some embodiments, the nucleic acid samples include at least a first nucleic acid sample and a second nucleic acid sample; the first nucleic acid sample and the second nucleic acid sample may have identical or different numbers of bases, and the first nucleic acid sample and the second nucleic acid sample each include a tag sequence at least at one end. The preset number of base extension cycles of the first nucleic acid sample includes M₁, and the preset number of base extension cycles of the second nucleic acid sample includes N₁, in which case outputting sequencing data to an external server at nodes according to preset numbers of base extension cycles, and outputting base calling results by the external server on the basis of the sequencing data may vary depending on the values of M₁ and N₁. In one embodiment, N₁ > M₁, in which case outputting sequencing data to an external server at nodes according to preset numbers of base extension cycles, and outputting base calling results by the external server on the basis of the sequencing data include:
after M₁ base extension cycles are completed for the first nucleic acid sample and the second nucleic acid sample and when base extension cycle P₁ of the second nucleic acid sample is ongoing or complete, outputting first sequencing data to the external server, and outputting a first base calling result by the external server on the basis of the first sequencing data, the first base calling result including base calling results correspondingly generated after M₁ base extension cycles are completed for the first nucleic acid sample and the second nucleic acid sample,
where M₁, N₁, and P₁ are all natural numbers, and N₁ > P₁ ≥ M₁.

After the process described above is completed, the base extension of the first nucleic acid sample and the second nucleic acid sample is continued, and when the next preset number of base extension cycles is achieved, base calling results are output again.

In some embodiments, the preset number of base extension cycles of the first nucleic acid sample is M₁, in which case after the process described above is completed, there is no need to continue to perform base extension reactions on the first nucleic acid sample, but the base extension reactions of the second nucleic acid sample are not stopped. In some embodiments, the preset number of base extension cycles of the first nucleic acid sample is M₁, and the preset number of base extension cycles of the second nucleic acid sample is N₁, in which case after the process described above is completed, the method further includes:
after the first base calling result is output and when N₁ base extension cycles are completed,
outputting a second base calling result.

Outputting base calling results in batches not only facilitates the earlier outputting of the base calling results of nucleic acid samples with relatively small preset numbers of base extension cycles, but also enables the data output in each base extension reaction period to be stored and backed up, thereby increasing the safety of the data.

In one embodiment, the preset number of base extension cycles of the first nucleic acid sample is consistent with its number of bases; that is, the number of bases of the first nucleic acid sample is M₁; and the preset number of base extension cycles of the second nucleic acid sample is consistent with its number of bases; that is, the number of bases of the second nucleic acid sample is N₁.

For the above-mentioned nucleic acid sequences for which base extension reactions are started simultaneously, the base extension reactions may be performed through the same sequencing system, or may be performed synchronously through different systems.

In a second possible embodiment, a plurality of nucleic acid samples are provided, and the numbers of base extension cycles performed for at least some of the nucleic acid samples are not synchronous. In this case, as one implementation, different sequencing systems are used to sequence the nucleic acid samples, and the times for the different sequencing systems to start base extension reactions are different. Certainly, when the same sequencing system is used to perform base extension on nucleic acid samples, controlling the reaction conditions between different reaction regions, such as different flow channels in a biochip, also enables the progress of the base extension reactions in the different reaction regions to be controllably adjusted. Similarly, each nucleic acid sample includes one tag sequence at one end, and the sequencing of the tag sequence is completed earlier than the sequencing of a sample sequence of the nucleic acid sample, so that base calling results of nucleic acid samples for which the preset numbers of base extension cycles have been achieved can be assigned or subjected to other analyses based on the tag sequences.

In some embodiments, the nucleic acid samples have identical or different preset numbers of base extension cycles. The "the nucleic acid samples have identical preset numbers of base extension cycles" means that all the nucleic acid samples have identical preset numbers of base extension cycles; however, as the times to start base extension reactions are different, the base extension reactions are not synchronous, such that the times required to complete sequencing of the nucleic acid samples for which base extension reactions are started at different times are different. The "the nucleic acid samples have different preset numbers of base extension cycles" includes: the case that each of the nucleic acid samples has a different preset number of base extension cycles, and further includes: the case that some of the nucleic acid samples have different preset numbers of base extension cycles. Additionally, for nucleic acid samples with identical preset numbers of base extension cycles, the times to start base extension reactions may be identical or different.

In this case, outputting sequencing data to an external server at nodes according to preset numbers of base extension cycles, and outputting base calling results by the external server on the basis of the sequencing data include:
in the order in which the preset numbers of base extension cycles occur, when the preset number of base extension cycles of each of the nucleic acid samples are completed, outputting sequencing data to the external server, and outputting the base calling results by the external server on the basis of the sequencing data, with base extension reactions of the nucleic acid samples the sequencing of which has not been completed being not stopped.

The method can be divided into several cases according to the number of times of the preset numbers of base extension cycles set for the nucleic acid samples.

In one implementation, one preset number of base extension cycles is set for each nucleic acid sample for which the time to start base extension reactions is uncertain, and the nucleic acid samples may have identical or different preset numbers of base extension cycles. In this case, in the order in which the preset numbers of base extension cycles are achieved, base calling results for a certain number of base extension cycles are sequentially output. Illustratively, base extension reactions are started on five nucleic acid samples, and the five nucleic acid samples all have a preset number of base extension cycles of C1, in which case in the process of the sequencing system performing base extension reactions on the five nucleic acid samples, in the order in which the five nucleic acid samples achieve C1, base calling results of the first cycle to cycle C1 are output when the number of base extension cycles of each of the samples reaches C1. Certainly, for a nucleic acid sample for which C1 is achieved later, base calling results of correspondingly completed base extension reactions may be output synchronously when base calling results of other nucleic acid samples for which C1 is achieved earlier are output, or base calling results of the first cycle to cycle C1 may be output in one step when C1 base extension cycles are completed for the sample.

In another implementation, two or more preset numbers of base extension cycles are set for at least some of the nucleic acid samples for which the time to start base extension reactions is uncertain. In this case, similarly, in the order in which the preset numbers of base extension cycles are achieved, base calling results for a certain number of base extension cycles are sequentially output. Illustratively, base extension reactions are started simultaneously for two nucleic acid samples; two preset number of base extension cycles, C1 and C3, are set for the first nucleic acid sample, and two preset numbers of base extension cycles, C2 and C4, are set for the second nucleic acid sample. In this case, in the order in which C1, C2, C3, and C4 are achieved, base calling results are output after each of the preset numbers of base extension cycles is achieved. Illustratively, when the preset numbers of base extension cycles are achieved in the order of C1 < C2 < C3 < C4: when the number of base extension cycles is C1, base calling results of the first cycle to cycle C1 of the first nucleic acid sample are output, and at the same time, the base extension reactions of the first nucleic acid sample and the second nucleic acid sample are not stopped; when the number of base extension cycles reaches C2, base calling results of the first cycle to cycle C1 of the second nucleic acid sample are output, and at the same time, the base extension reactions of the first nucleic acid sample and the second nucleic acid sample are not stopped; when the number of base extension cycles reaches C3, base calling results of cycle C2 to cycle C3 of the first nucleic acid sample are output, and at the same time, the base extension reactions of the second nucleic acid sample are not stopped; and when the number of base extension cycles of the second nucleic acid sample reaches C4, base calling results of cycle C3 to cycle C4 of the second nucleic acid sample are output. Certainly, base calling results of the first nucleic acid sample and the second nucleic acid sample may be output at nodes when each of the preset numbers of base extension cycles is achieved. For example, when the preset numbers of base extension cycles are achieved in the order of C1 < C2 < C3 < C4: when the number of base extension cycles is C1, base calling results of the first cycle to cycle C1 of the first nucleic acid sample and the second nucleic acid sample are output, and at the same time, the base extension reactions of the first nucleic acid sample and the second nucleic acid sample are not stopped; when the number of base extension cycles reaches C2, base calling results of cycle C1 to cycle C2 of the first nucleic acid sample and the second nucleic acid sample are output, and at the same time, the base extension reactions of the first nucleic acid sample and the second nucleic acid sample are not stopped; when the number of base extension cycles reaches C3, base calling results of cycle C2 to cycle C3 of the first nucleic acid sample and the second nucleic acid sample are output, and at the same time, the base extension reactions of the second nucleic acid sample are not stopped; and when the number of base extension cycles of the second nucleic acid sample reaches C4, base calling results of cycle C3 to cycle C4 of the second nucleic acid sample are output.

The base calling results for a certain number of base extension cycles refer to base calling results corresponding to the base extension reactions from the first base extension cycle started after the previous preset number of base extension cycles ends to the preset number of base extension cycles. When the current preset number of base extension cycles is the minimum value, for example, C1, base calling results of the base extension reactions from the first base extension cycle to the preset number of base extension cycles, for example, from the first cycle to cycle C1, are output. Similarly, for a nucleic acid sample for which a preset number of base extension cycles has not been reached, corresponding base calling results may be output when preset numbers of base extension cycles of other nucleic acid samples are achieved; for example, for a nucleic acid sample with preset numbers of base extension cycles of C2 and C4, base calling results may be output at C1; alternatively, base calling results may not be output when preset numbers of base extension cycles of other samples are achieved, and instead, all base calling samples are output when the nucleic acid sample's own preset numbers of base extension cycles are achieved; for example, for a nucleic acid sample with preset numbers of base extension cycles of C2 and C4, base calling results are not output when the number of base extension cycles reaches C1, but when the number of base extension cycles reaches C2 or C4, base calling results of the nucleic acid sample are sequentially output.

Similarly, in this implementation, the preset numbers of base extension cycles of the nucleic acid sample may be set in advance. For a nucleic acid sample containing a plurality of preset numbers of base extension cycles, the first preset number of base extension cycles may be set in advance, or set based on the preset numbers of base extension cycles of other nucleic acid samples that occur earlier than the first preset number of base extension cycles; the second preset number of base extension cycles or later preset number(s) of base extension cycles may be all set in advance, or set based on the base calling results corresponding to the first preset number of base extension cycles. Illustratively, when a nucleic acid sample includes a first preset number of base extension cycles, and when base calling result data corresponding to the first preset number of base extension cycles are insufficient to achieve its application purpose or its application effect, a second preset number of base extension cycles may be set on the basis of the first preset number of base extension cycles to collect the base calling results of the sequencing cycles between the first preset number of base extension cycles and the second preset number of base extension cycles for further analysis until its application purpose or the desired effect is achieved.

For some embodiments, some nucleic acid samples have a preset number of base extension cycles that is equal to the number of bases of the nucleic acid samples. The number of bases of the nucleic acid samples refers to the number of bases constituting the nucleic acid samples, and may also refer to the number of nucleotides forming the nucleic acid molecules. In this case, outputting base calling results after the preset numbers of base extension cycles are completed for some of the nucleic acid samples enables the base calling results of these nucleic acid samples to be output in advance without interference before the sequencing of other nucleic acid samples has not been completed, thereby improving the overall sequencing efficiency. In this case, similarly, in the order in which the preset numbers of base extension cycles of the nucleic acid samples are achieved, base calling results of the current node are output when the preset number of base extension cycles are completed for each of the nucleic acid samples.

It should be understood that the base calling output results of each node may be output after the current preset number of base extension cycles is achieved and before the next preset number of base extension cycles is achieved.

In some embodiments, the nucleic acid samples include at least a third nucleic acid sample and a fourth nucleic acid sample; the third nucleic acid sample and the fourth nucleic acid sample may have identical or different numbers of bases, and the third nucleic acid sample and the fourth nucleic acid sample each include a tag sequence at least at one end. In one embodiment, the preset number of base extension cycles of the third nucleic acid sample is M₂, and the preset number of base extension cycles of the fourth nucleic acid sample is N₂, where N₂ = M₂, and base extension reactions are not simultaneously started for the third nucleic acid sample and the fourth nucleic acid sample, in which case base calling results are output at nodes in the order in which the preset numbers of base extension cycles occur. In one embodiment, outputting sequencing data to an external server at nodes according to preset numbers of base extension cycles, and outputting base calling results by the external server on the basis of the sequencing data include:
when P₂ base extension cycles are completed for the third nucleic acid sample and P₃ base extension cycles are completed for the fourth nucleic acid sample, outputting a third base calling result, where M₂, N₂, and P₂ are all natural numbers.

When P₂ = M₂ > P₃, the third nucleic acid sample undergoes base extension reactions earlier than the fourth nucleic acid sample, and the third base calling result includes base calling results correspondingly generated after M₂ base extension cycles are completed for the third nucleic acid sample, or the third base calling result includes base calling results correspondingly generated after M₂ base extension cycles are completed for the third nucleic acid sample and base calling results correspondingly generated after P₃ base extension cycles are completed for the fourth nucleic acid sample. After the process described above is completed, the base extension of the fourth nucleic acid sample is continued, and when the next preset number of base extension cycles is achieved, base calling results are output again. That is, after the third base calling result is output, and after N₂ base extension cycles are completed for the fourth nucleic acid sample, a fourth base calling result is output.

When P₃ = N₂ > P₂, the third nucleic acid sample undergoes base extension reactions later than the fourth nucleic acid sample, and the third base calling result includes base calling results correspondingly generated after N₂ base extension cycles are completed for the fourth nucleic acid sample, or the third base calling result includes base calling results correspondingly generated after N₂ base extension cycles are completed for the fourth nucleic acid sample and base calling results correspondingly generated after P₂ base extension cycles are completed for the third nucleic acid sample. After the process described above is completed, the base extension of the third nucleic acid sample is continued, and when the next preset number of base extension cycles is achieved, base calling results are output again. That is, after the third base calling result is output, and after N₂ base extension cycles are completed for the third nucleic acid sample, a fourth base calling result is output.

Outputting base calling results in batches in this manner facilitates the earlier outputting of the base calling results of nucleic acid samples with relatively small preset numbers of base extension cycles, and also enables the data output in each base extension reaction period to be stored and backed up, thereby increasing the safety of the data.

In one embodiment, the preset number of base extension cycles of the third nucleic acid sample is consistent with its number of bases; that is, the number of bases of the third nucleic acid sample is M₂; and the preset number of base extension cycles of the fourth nucleic acid sample is consistent with its number of bases; that is, the number of bases of the fourth nucleic acid sample is N₂.

In one embodiment, the same nucleic acid template includes a fifth nucleic acid sample and a sixth nucleic acid sample; the fifth nucleic acid sample and the sixth nucleic acid sample each include a sequence region and, at an end, a tag region, and the fifth nucleic acid sample and the sixth nucleic acid sample are connected by the tag regions thereof. In this embodiment, in the step of performing base extension reactions on a plurality of nucleic acid samples, base extension reactions are performed on the tag regions of the fifth nucleic acid sample and the sixth nucleic acid sample, and then base extension reactions are performed on the sequence regions of the fifth nucleic acid sample and the sixth nucleic acid sample.

In an embodiment of the present application, outputting base calling results at nodes may be achieved by outputting the base calling results to an external server. The external server refers to a server other than a server of an apparatus where the sequencing system is present, and the external server and the sequencing system need to be connected through communication. In some embodiments, software for base calling may be changed so that base calling results are completed at a device end where the sequencing system is present while the sequencing is not interrupted. It should be understood that in the embodiments of the present application, the base calling results of the base extension reactions performed using a plurality of different sequencing systems can be achieved through one public external server.

As one embodiment, the sequencing system includes a first sequencing system. In this case, outputting sequencing data to an external server at nodes includes:
S 100: in the case that the base extension reactions of the first sequencing system are determined to have reached a first preset number of base extension cycles and a sequencing run is determined to have not yet been finished, connecting the first sequencing system and one or more specified external servers, starting transmission of at least part of the sequencing data to the external servers, and starting monitoring of the transmission process.

In this step, the base extension reactions of the first sequencing system mean using the first sequencing system to perform base extension reactions on nucleic acid samples. The first preset number of base extension cycles refers to a preset number of base extension cycles achieved first in the process of using the first sequencing system to perform base extension reactions on nucleic acid samples. In this case, the first sequencing system and one or more specified external servers are connected, transmission of at least part of the sequencing data to the external servers is started, and monitoring of the transmission process is started, and the obtained sequencing data has been transmitted to the external servers. In this case, the sequencing data includes first sequencing data, which are signals generated by using the first sequencing system to perform base extension reaction runs until the first preset number of base extension cycles is reached. For a nucleic acid sample for which no first preset number of base extension cycles is set, the first sequencing data may not include signals generated by performing base extension reaction runs for these nucleic acid samples until the first preset number of base extension cycles is reached, but may be output in one step after a preset number of base extension cycles occurs for the nucleic acid sample for the first time. In an embodiment of the present application, the signals generated from the number of base extension cycles refer to characteristic changes that are generated during base extension reactions, formed due to characteristic alteration, and can be recognized in some way, such as fluorescence signals, electrochemical signals, voltage signals, and current signals.

In an embodiment of the present application, the external servers refer to sequencing servers different from the sequencing server of the sequencing system. The external servers may be arranged in the same apparatus as the sequencing system or in an apparatus different from where the sequencing system is arranged, so that cloud data transmission is realized.

In the embodiments of the present application, the transmission process is monitored to acquire the progress of data transmission and whether the transmission process is abnormal or not.

In some embodiments, in the process of using the first sequencing system to perform base extension reactions on nucleic acid samples, when the nucleic acid samples include a second preset number of base extension cycles, and after the base extension reactions of the first sequencing system are determined to have reached the first preset number of base extension cycles, the sequencing of the nucleic acid samples is continued, and the second preset number of base extension cycles is achieved. In this case, after S 100, the method further includes:
S200: when the second preset number of base extension cycles is determined to have been reached,
starting the transmission of at least part of the sequencing data to the external servers.

For one implementation, the sequencing data include second sequencing data, which are signals generated by using the first sequencing system to perform base extension reaction runs until the second preset number of base extension cycles is reached from the first preset number of base extension cycles. That is, the sequencing data include: the corresponding sequencing data between the base extension reaction started after the first preset number of base extension cycles is achieved and the base extension reaction when the second preset number of base extension cycles is achieved.

For another implementation, if the second preset number of base extension cycles is the first preset number of base extension cycles set for nucleic acid samples, then, for these nucleic acid samples, the second sequencing data are signals generated from the first base extension cycle to the point when the second preset number of base extension cycles is reached. That is, the sequencing data include second sequencing data, which are signals generated by using the first sequencing system to perform base extension reaction runs until the second preset number of base extension cycles is reached from the first preset number of base extension cycles.

In some embodiments, S200 further includes:
in the case that the transmission of the first sequencing data has not yet been finished, starting monitoring of a transmission process of the second sequencing data to the external servers.

A data transmission state is acquired through monitoring, and after the transmission of the first sequencing data is finished, the transmission progress of the second sequencing data and information on whether the transmission is abnormal or not are monitored.

In one implementation, the sequencing system further includes a second sequencing system, and the step of performing base extension reactions on nucleic acid samples further includes: using the second sequencing system to perform base extension reactions to generate sequencing data;
in this case, after S 100, the method further includes:
S300: in the case that the base extension reactions of the second sequencing system are determined to have reached a third preset number of base extension cycles and a sequencing run is determined to have not yet been finished, connecting the second sequencing system and one or more specified external servers, starting transmission of at least part of the sequencing data to the external servers, and starting monitoring of the transmission process,
the sequencing data including third sequencing data, which are signals generated by using the second sequencing system to perform base extension reaction runs until the third preset number of base extension cycles is reached.

It can be understood that the first sequencing system and the second sequencing system each independently perform base extension reactions on nucleic acid samples, and the third preset number of base extension cycles is to the second sequencing system as the first preset number of base extension cycles is to the first sequencing system. Therefore, for the sake of brevity, no further description is provided herein.

In an embodiment of the present application, before starting the transmission of at least part of the sequencing data to the servers, S 100 further includes:
S 102: configuring at least part of the sequencing data, including computing a first hash value that uniquely corresponds to the at least part of the sequencing data.

Before the transmission of at least part of the sequencing data to the servers, at least part of the sequencing data is configured. The configuration process includes computing a first hash value that uniquely corresponds to the at least part of the sequencing data. The first hash value refers to a value generated by processing the at least part of the sequencing data through a specific hash function. Inputting different sequencing data will output different first hash values.

In some embodiments, starting the transmission of at least part of the sequencing data to the servers includes starting transmission of the first hash value to the servers, or S 100 further includes S104: transmitting the first hash value to the servers.

The first hash value that uniquely corresponds to the at least part of the sequencing data is configured first, and when the at least part of the sequencing data is transmitted to the servers, the first hash value configured first is then transmitted to the servers at the same time. Alternatively, the first hash value that uniquely corresponds to the at least part of the sequencing data is configured first, and after the sequencing data is transmitted to the servers, the configured first hash value is then transmitted to the servers.

In some embodiments, after S 100, the method further includes:
S106: computing a second hash value that uniquely corresponds to the sequencing data transmitted to the servers, optionally, the first hash value and/or the second hash value being MD5 value(s); and
S108: comparing the first hash value and the second hash value on the servers, determining that the transmission of the sequencing data has been finished if the first hash value and the second hash value are consistent, and determining that the transmission of the sequencing data has not yet been finished if the first hash value and the second hash value are inconsistent.

After the at least part of the sequencing data is transmitted to the servers, a second hash value that uniquely corresponds to the sequencing data transmitted to the servers is computed, and the first hash value received is then compared with the second hash value: if the first hash value and the second hash value are consistent, it means that the sequencing data that need to be transmitted to the servers are consistent with the sequencing data that have been transmitted to the servers, and then the transmission of the sequencing data is determined to have been finished; if the first hash value and the second hash value are not consistent, it means that the sequencing data that need to be transmitted to the servers are not consistent with the sequencing data that have been transmitted to the servers, and then the transmission of the sequencing data is determined to have not yet been finished.

The first hash value and/or the second hash value may be MD5 value(s). The MD5 values may be obtained by computation through the MD5 message digest algorithm, which is a widely used cryptographic hash function. The computation yields a 128-bit (16-byte) hash value for ensuring the integrity and consistency of message transmission.

In an embodiment of the present application, to realize effective transmission of data, after S 100, the method further includes:
S400: in the case that the transmission of the sequencing data is determined to have not yet been finished and a connection between the sequencing system and the external servers is determined to be in an abnormal state, disconnecting the sequencing system and the external servers.

In an embodiment of the present application, the abnormal state includes at least one of the following cases:
(a) the transmission of the sequencing data is slower than a preset transmission speed;
(b) the transmission of the sequencing data is interrupted; and
(c) the external servers have insufficient operational space and/or physical space.

When a connection between the sequencing system and the external servers has at least any one of the abnormalities described above, the sequencing system and the external servers are disconnected. In some embodiments, when the abnormal state includes case (a), after disconnecting the sequencing system and the external servers, S400 further includes:
restarting the transmission of the sequencing data to the external servers within a preset time and continuing the monitoring of the transmission process.

In some embodiments, a criterion for deciding that the abnormal state includes case (b) is that: within a preset time, the amount of data exchange is 0.

In some embodiments, when the abnormal state includes case (b), after disconnecting the sequencing system and the external servers, S400 further includes:
active terminal network communication, restarting network communication after waiting for the preset time, restarting the transmission of the sequencing data to the external servers, and continuing the monitoring of the transmission process.

In some embodiments, the sequencing system for performing base extension reactions on nucleic acid samples includes first data transmission software. In this case, starting transmission of at least part of the sequencing data to the external servers includes:
acquiring a sequencing intermediate data packet by the first data transmission software, the sequencing intermediate data packet including at least nucleic acid sample information derived from the sequencing system and optical signal information generated by the base extension reactions; and
transmitting the sequencing intermediate data packet to the external servers by the first data transmission software through a communication network;
determining the base calling results by the external servers on the basis of the sequencing data includes:
   completing base calling by the external servers on the basis of the sequencing intermediate data packet, and saving the obtained base calling results to a local folder.

In some embodiments, the sequencing system further includes sequencing software and a DC Server, and the external servers include second data transmission software that receives the sequencing intermediate data packet from the first data transmission software.

In some embodiments, acquiring a sequencing intermediate data packet includes:
acquiring the nucleic acid sample information from the sequencing software by the first data transmission software, and
acquiring the optical signal information generated by the base extension reactions from the DC Server by the first data transmission software.

In an embodiment of the present application, after any one of the steps described above is completed, the following step may be performed:
S500: in the case that the transmission of the sequencing data is determined to have been finished, disconnecting the sequencing system and the external servers and ending the monitoring of the corresponding transmission process.

The above description is only for the purpose of illustrating the preferred embodiments of the present application, and is not intended to limit the scope of the present application. Any modification, equivalent, improvement, and the like made without departing from the spirit and principle of the present application shall fall within the protection scope of the present application.

The present invention may be defined by the following items.

Item 1: A sequencing method, comprising the following steps: using a sequencing system to perform base extension reactions on nucleic acid samples; and outputting sequencing data to an external server at nodes according to preset numbers of base extension cycles, and outputting base calling results by the external server on the basis of the sequencing data.

Item 2: The method according to item 1, wherein a plurality of nucleic acid samples are provided; the preset numbers of base extension cycles are numbers of base extension cycles required for predetermined outputs of the nucleic acid samples, and at least some of the nucleic acid samples have different preset numbers of base extension cycles.

Item 3: The method according to item 2, wherein base extension reactions are started simultaneously for at least some of the nucleic acid samples.

Item 4: The method according to item 3, wherein outputting sequencing data to an external server at nodes according to preset numbers of base extension cycles, and outputting base calling results by the external server on the basis of the sequencing data comprise: in an order in which the preset numbers of base extension cycles occur, when the preset number of base extension cycles are completed for each of the nucleic acid samples, outputting sequencing data of a current node to the external server, and outputting the base calling results by the external server on the basis of the sequencing data, with base extension reactions of the nucleic acid samples, sequencing of which has not been completed, being not stopped,
wherein each of the nucleic acid samples comprises one tag sequence at one end.

Item 5: The method according to item 4, wherein some of the nucleic acid samples have a preset number of base extension cycles that is equal to a number of bases of the nucleic acid samples.

Item 6: The method according to item 5, wherein outputting sequencing data to an external server at nodes according to preset numbers of base extension cycles, and outputting base calling results by the external server on the basis of the sequencing data comprise: in ascending order according to numbers of bases, sequentially completing sequencing of the nucleic acid samples, and in a final base extension cycle of the nucleic acid sample corresponding to each of the numbers of bases and when base extension reactions of the nucleic acid samples, sequencing of which has not been completed, are ongoing, outputting the sequencing data to the external server; and outputting the base calling results by the external server on the basis of the sequencing data.

Item 7: The method according to any one of items 2-6, wherein the nucleic acid samples comprise at least a first nucleic acid sample and a second nucleic acid sample; the first nucleic acid sample and the second nucleic acid sample have identical or different numbers of bases, and the first nucleic acid sample and the second nucleic acid sample each comprise a tag sequence at least at one end.

Item 8: The method according to item 7, wherein a preset number of base extension cycles of the first nucleic acid sample comprises M₁, and a preset number of base extension cycles of the second nucleic acid sample comprises N₁; outputting sequencing data to an external server at nodes according to preset numbers of base extension cycles, and outputting base calling results by the external server on the basis of the sequencing data comprise: after M1 base extension cycles are completed for the first nucleic acid sample and the second nucleic acid sample and when base extension cycle P₁ of the second nucleic acid sample is ongoing or complete, outputting first sequencing data to the external server, and outputting a first base calling result by the external server on the basis of the first sequencing data, the first base calling result comprising base calling results correspondingly generated after M₁ base extension cycles are completed for the first nucleic acid sample and the second nucleic acid sample, wherein M₁, N₁, and P₁ are all natural numbers, and N₁ > P₁ ≥ M₁.

Item 9: The method according to item 8, further comprising: after the first base calling result is output and when N₁ base extension cycles are completed, outputting a second base calling result. Item 10: The method according to item 8 or 9, wherein a number of bases of the first nucleic acid sample is M₁, and a number of bases of the second nucleic acid sample is N₁.

Item 11: The method according to any one of items 1-10, wherein a plurality of nucleic acid samples are provided, and numbers of base extension cycles performed for at least some of the nucleic acid samples are not synchronous.

Item 12: The method according to item 11, wherein the nucleic acid samples have identical or different preset numbers of base extension cycles.

Item 13: The method according to item 12, wherein outputting sequencing data to an external server at nodes according to preset numbers of base extension cycles, and outputting base calling results by the external server on the basis of the sequencing data comprise: in an order in which the preset numbers of base extension cycles occur, when the preset number of base extension cycles of each of the nucleic acid samples are completed, outputting the sequencing data to the external server, and outputting the base calling results by the external server on the basis of the sequencing data, with base extension reactions of the nucleic acid samples, sequencing of which has not been completed, being not stopped, wherein each of the nucleic acid samples comprises a tag sequence at one end. Item 14: The method according to item 13, wherein at least some of the nucleic acid samples have a preset number of base extension cycles that is equal to a number of bases of the nucleic acid samples.

Item 15: The method according to any one of items 11-14, wherein the nucleic acid samples comprise at least a third nucleic acid sample and a fourth nucleic acid sample, and the third nucleic acid sample and the fourth nucleic acid sample each comprise a tag sequence at least at one end. Item 16: The method according to item 15, wherein a preset number of base extension cycles of the third nucleic acid sample is M₂, and a preset number of base extension cycles of the fourth nucleic acid sample is N₂; outputting sequencing data to an external server at nodes according to preset numbers of base extension cycles, and outputting base calling results by the external server on the basis of the sequencing data comprise: when P₂ base extension cycles are completed for the third nucleic acid sample and P₃ base extension cycles are completed for the fourth nucleic acid sample, outputting a third base calling result, the third base calling result comprising base calling results correspondingly generated after M₂ base extension cycles are completed for the third nucleic acid sample and base calling results correspondingly generated after P₃ base extension cycles are completed for the fourth nucleic acid sample, wherein M₂, N₂, P₂, and P₃ are all natural numbers, and N₂ = M₂.

Item 17: The method according to item 16, wherein the third nucleic acid sample undergoes base extension reactions earlier than the fourth nucleic acid sample, and P₂ = M₂ > P₃; the third base calling result comprises base calling results correspondingly generated after M₂ base extension cycles are completed for the third nucleic acid sample, or the third base calling result comprises base calling results correspondingly generated after M₂ base extension cycles are completed for the third nucleic acid sample, and base calling results correspondingly generated after P₃ base extension cycles are completed for the fourth nucleic acid sample.

Item 18: The method according to item 16, wherein the third nucleic acid sample undergoes base extension reactions later than the fourth nucleic acid sample, and P₃ = N₂ > P₂; the third base calling result comprises base calling results correspondingly generated after N₂ base extension cycles are completed for the fourth nucleic acid sample, or the third base calling result comprises base calling results correspondingly generated after N₂ base extension cycles are completed for the fourth nucleic acid sample, and base calling results correspondingly generated after P₂ base extension cycles are completed for the third nucleic acid sample.

Item 19: The method according to item 17, wherein after the third base calling result is output and after the preset numbers of base extension cycles are achieved for both nucleic acid samples, a fourth base calling result is output.

Item 20: The method according to any one of items 16-19, wherein a number of bases of the third nucleic acid sample is M₂, and a number of bases of the fourth nucleic acid sample is N₂.

Item 21: The method according to any one of items 7-10 and 13-20, wherein in the nucleic acid samples, the tag sequences are sequenced earlier than other sequences of the nucleic acid samples.

Item 22: The method according to any one of items 1-21, wherein a plurality of nucleic acid samples are provided, and the nucleic acid samples are DNA samples and/or RNA samples from different sources.

Item 23: The method according to any one of items 1-21, wherein a plurality of nucleic acid samples are provided, each in a different nucleic acid template.

Item 24: The method according to any one of items 1-21, wherein a plurality of nucleic acid samples are provided, and two or more of the nucleic acid samples are in the same nucleic acid template.

Item 25: The method according to item 24, wherein the same nucleic acid template comprises a fifth nucleic acid sample and a sixth nucleic acid sample; the fifth nucleic acid sample and the sixth nucleic acid sample each comprise a sequence region and, at an end, a tag region, and the fifth nucleic acid sample and the sixth nucleic acid sample are connected by the tag regions thereof. Item 26: The method according to item 25, wherein in a step of performing base extension reactions on a plurality of nucleic acid samples,
base extension reactions are performed on the tag regions of the fifth nucleic acid sample and the sixth nucleic acid sample, and then base extension reactions are performed on the sequence regions of the fifth nucleic acid sample and the sixth nucleic acid sample.

Item 27: The method according to any one of items 23-26, wherein the nucleic acid samples are fixed to a surface of a chip.

Item 28: The method according to item 27, wherein a plurality of liquid flow channels are arranged on the surface of the chip, and nucleic acid templates derived from different libraries are arranged in different channels, respectively.

Item 29: The method according to any one of items 1-28, wherein the sequencing system comprises a first sequencing system; outputting sequencing data to an external server at nodes comprises: S100: in the case that base extension reactions of the first sequencing system are determined to have reached a first preset number of base extension cycles and a sequencing run is determined to have not yet been finished, connecting the first sequencing system and one or more specified external servers, starting transmission of at least part of the sequencing data to the external servers, and starting monitoring of a transmission process, the sequencing data comprising first sequencing data, which are signals generated by using the first sequencing system to perform base extension reaction runs until the first preset number of base extension cycles is reached; and S500: in the case that transmission of the sequencing data is determined to have been finished, disconnecting the sequencing system and the external servers and ending the monitoring of the corresponding transmission process.

Item 30: The method according to item 29, further comprising, after S100, S200: when a second preset number of base extension cycles is determined to have been reached, starting the transmission of at least part of the sequencing data to the external servers, the sequencing data comprising second sequencing data, which are signals generated by using the first sequencing system to perform base extension reaction runs until the second preset number of base extension cycles is reached from the first preset number of base extension cycles.

Item 31: The method according to item 30, wherein S200 further comprises: in the case that transmission of the first sequencing data has not yet been finished, starting monitoring of a transmission process of the second sequencing data to the external servers.

Item 32: The method according to any one of items 29-31, wherein the sequencing system further comprises a second sequencing system; the step of performing base extension reactions on nucleic acid samples further comprises: using the second sequencing system to perform the base extension reactions to generate sequencing data; after S100, the method further comprises: S300: in the case that the base extension reactions of the second sequencing system are determined to have reached a third preset number of base extension cycles and a sequencing run is determined to have not yet been finished, connecting the second sequencing system and one or more specified external servers, starting transmission of at least part of the sequencing data to the external servers, and starting monitoring of a transmission process, the sequencing data comprising third sequencing data, which are signals generated by using the second sequencing system to perform base extension reaction runs until the third preset number of base extension cycles is reached.

Item 33: The method according to any one of items 29-32, wherein before starting the transmission of at least part of the sequencing data to the servers, S100 further comprises: S102: configuring at least part of the sequencing data, including computing a first hash value that uniquely corresponds to the at least part of the sequencing data.

Item 34: The method according to item 33, wherein starting the transmission of at least part of the sequencing data to the servers comprises starting transmission of the first hash value to the servers, or S100 further comprises S104: transmitting the first hash value to the servers.

Item 35: The method according to item 34, further comprising, after S100, S106: computing a second hash value that uniquely corresponds to the sequencing data transmitted to the servers, optionally, the first hash value and/or the second hash value being MD5 value(s); and S108: comparing the first hash value and the second hash value on the servers, determining that the transmission of the sequencing data has been finished if the first hash value and the second hash value are consistent, and determining that the transmission of the sequencing data has not yet been finished if the first hash value and the second hash value are inconsistent.

Item 36: The method according to any one of items 29-35, wherein the sequencing system comprises first data transmission software; starting transmission of at least part of the sequencing data to the external servers comprises: acquiring a sequencing intermediate data packet by the first data transmission software, the sequencing intermediate data packet comprising at least nucleic acid sample information derived from the sequencing systems and optical signal information generated by the base extension reactions; and transmitting the sequencing intermediate data packet to the external servers by the first data transmission software through a communication network; determining the base calling results by the external servers on the basis of the sequencing data comprises: completing base calling by the external servers on the basis of the sequencing intermediate data packet, and saving the obtained base calling results to a local folder.

Item 37: The method according to item 36, wherein the sequencing system further comprises sequencing software and a DC Server, and the external servers comprise second data transmission software that receives the sequencing intermediate data packet from the first data transmission software.

Item 38: The method according to item 37, wherein acquiring a sequencing intermediate data packet comprises: acquiring the nucleic acid sample information from the sequencing software by the first data transmission software, and acquiring the optical signal information generated by the base extension reactions from the DC Server by the first data transmission software.

Item 39: The method according to any one of items 29-38, further comprising, after S100, S400: in the case that transmission of the sequencing data is determined to have not yet been finished and a connection between the sequencing system and the external servers is determined to be in an abnormal state, disconnecting the sequencing system and the external servers.

Item 40: The method according to any one of items 29-39, wherein the abnormal state comprises at least one of the following cases:
(a) the transmission of the sequencing data is slower than a preset transmission speed;
(b) the transmission of the sequencing data is interrupted; and
(c) the external servers have insufficient operational space and/or physical space.

Item 41: The method according to item 40, wherein when the abnormal state comprises case (a), after disconnecting the sequencing system and the external servers, S400 further comprises: restarting the transmission of the sequencing data to the external servers within a preset time and continuing the monitoring of the transmission process.

Item 42: The method according to item 40, wherein a criterion for deciding that the abnormal state comprises case (b) is that: within a preset time, an amount of data exchange is 0.

Item 43: The method according to item 40 or 42, wherein when the abnormal state comprises case (b), after disconnecting the sequencing system and the external servers, S400 further comprises: active terminal network communication, restarting network communication after waiting for the preset time, restarting the transmission of the sequencing data to the external servers, and continuing the monitoring of the transmission process.

## Claims

1. A sequencing method, comprising the following steps:
using a sequencing system to perform base extension reactions on nucleic acid samples; and
outputting sequencing data to an external server at nodes according to preset numbers of base extension cycles, and outputting base calling results by the external server on the basis of the sequencing data.

2. The method according to claim 1, wherein a plurality of nucleic acid samples are provided; the preset numbers of base extension cycles are numbers of base extension cycles required for predetermined outputs of the nucleic acid samples, and at least some of the nucleic acid samples have different preset numbers of base extension cycles;
optionally, base extension reactions are started simultaneously for at least some of the nucleic acid samples;
optionally, outputting sequencing data to an external server at nodes according to preset numbers of base extension cycles, and outputting base calling results by the external server on the basis of the sequencing data comprise:
in an order in which the preset numbers of base extension cycles occur, when the preset number of base extension cycles are completed for each of the nucleic acid samples, outputting sequencing data of a current node to the external server, and outputting the base calling results by the external server on the basis of the sequencing data, with base extension reactions of the nucleic acid samples, sequencing of which has not been completed, being not stopped,
wherein each of the nucleic acid samples comprises one tag sequence at one end;
optionally, some of the nucleic acid samples have a preset number of base extension cycles that is equal to a number of bases of the nucleic acid samples;
optionally, outputting sequencing data to an external server at nodes according to preset numbers of base extension cycles, and outputting base calling results by the external server on the basis of the sequencing data comprise:
in ascending order according to numbers of bases, sequentially completing sequencing of the nucleic acid samples, and in a final base extension cycle of the nucleic acid sample corresponding to each of the numbers of bases and when base extension reactions of the nucleic acid samples, sequencing of which has not been completed, are ongoing, outputting the sequencing data to the external server; and outputting the base calling results by the external server on the basis of the sequencing data.

3. The method according to claim 2, wherein the nucleic acid samples comprise at least a first nucleic acid sample and a second nucleic acid sample; the first nucleic acid sample and the second nucleic acid sample have identical or different numbers of bases, and the first nucleic acid sample and the second nucleic acid sample each comprise a tag sequence at least at one end;
optionally, a preset number of base extension cycles of the first nucleic acid sample comprises M₁, and a preset number of base extension cycles of the second nucleic acid sample comprises N₁; outputting sequencing data to an external server at nodes according to preset numbers of base extension cycles, and outputting base calling results by the external server on the basis of the sequencing data comprise:
after M1 base extension cycles are completed for the first nucleic acid sample and the second nucleic acid sample and when base extension cycle P₁ of the second nucleic acid sample is ongoing or complete, outputting first sequencing data to the external server, and outputting a first base calling result by the external server on the basis of the first sequencing data, the first base calling result comprising base calling results correspondingly generated after M₁ base extension cycles are completed for the first nucleic acid sample and the second nucleic acid sample,
wherein M₁, N₁, and P₁ are all natural numbers, and N₁ > P₁ ≥ M₁;
optionally, further comprising:
after the first base calling result is output and when N₁ base extension cycles are completed, outputting a second base calling result;
optionally, a number of bases of the first nucleic acid sample is M₁, and a number of bases of the second nucleic acid sample is N₁.

4. The method according to any one of claims 1-3, wherein a plurality of nucleic acid samples are provided, and numbers of base extension cycles performed for at least some of the nucleic acid samples are not synchronous;
optionally, the nucleic acid samples have identical or different preset numbers of base extension cycles;
optionally, outputting sequencing data to an external server at nodes according to preset numbers of base extension cycles, and outputting base calling results by the external server on the basis of the sequencing data comprise:
in an order in which the preset numbers of base extension cycles occur, when the preset number of base extension cycles of each of the nucleic acid samples are completed, outputting the sequencing data to the external server, and outputting the base calling results by the external server on the basis of the sequencing data, with base extension reactions of the nucleic acid samples, sequencing of which has not been completed, being not stopped,
wherein each of the nucleic acid samples comprises a tag sequence at one end;
optionally, at least some of the nucleic acid samples have a preset number of base extension cycles that is equal to a number of bases of the nucleic acid samples.

5. The method according to claim 4, wherein the nucleic acid samples comprise at least a third nucleic acid sample and a fourth nucleic acid sample, and the third nucleic acid sample and the fourth nucleic acid sample each comprise a tag sequence at least at one end;
optionally, a preset number of base extension cycles of the third nucleic acid sample is M₂, and a preset number of base extension cycles of the fourth nucleic acid sample is N₂;
outputting sequencing data to an external server at nodes according to preset numbers of base extension cycles, and outputting base calling results by the external server on the basis of the sequencing data comprise:
when P₂ base extension cycles are completed for the third nucleic acid sample and P₃ base extension cycles are completed for the fourth nucleic acid sample, outputting a third base calling result, the third base calling result comprising base calling results correspondingly generated after M₂ base extension cycles are completed for the third nucleic acid sample and base calling results correspondingly generated after P₃ base extension cycles are completed for the fourth nucleic acid sample,
wherein M₂, N₂, P₂, and P₃ are all natural numbers, and N₂ = M₂;
optionally, the third nucleic acid sample undergoes base extension reactions earlier than the fourth nucleic acid sample, and P₂ = M₂ > P₃;
the third base calling result comprises base calling results correspondingly generated after M₂ base extension cycles are completed for the third nucleic acid sample, or
the third base calling result comprises base calling results correspondingly generated after M₂ base extension cycles are completed for the third nucleic acid sample, and base calling results correspondingly generated after P₃ base extension cycles are completed for the fourth nucleic acid sample;
optionally, the third nucleic acid sample undergoes base extension reactions later than the fourth nucleic acid sample, and P₃ = N₂ > P₂;
the third base calling result comprises base calling results correspondingly generated after N₂ base extension cycles are completed for the fourth nucleic acid sample, or
the third base calling result comprises base calling results correspondingly generated after N₂ base extension cycles are completed for the fourth nucleic acid sample, and base calling results correspondingly generated after P₂ base extension cycles are completed for the third nucleic acid sample;
optionally, after the third base calling result is output and after the preset numbers of base extension cycles are achieved for both nucleic acid samples, a fourth base calling result is output;
optionally, a number of bases of the third nucleic acid sample is M₂, and a number of bases of the fourth nucleic acid sample is N₂.

6. The method according to claim 3, wherein in the nucleic acid samples, the tag sequences are sequenced earlier than other sequences of the nucleic acid samples.

7. The method according to any one of claims 1-6, wherein a plurality of nucleic acid samples are provided, and the nucleic acid samples are DNA samples and/or RNA samples from different sources;
optionally, a plurality of nucleic acid samples are provided, each in a different nucleic acid template.

8. The method according to any one of claims 1-7, wherein a plurality of nucleic acid samples are provided, and two or more of the nucleic acid samples are in the same nucleic acid template; optionally, the same nucleic acid template comprises a fifth nucleic acid sample and a sixth nucleic acid sample; the fifth nucleic acid sample and the sixth nucleic acid sample each comprise a sequence region and, at an end, a tag region, and the fifth nucleic acid sample and the sixth nucleic acid sample are connected by the tag regions thereof;
optionally, in a step of performing base extension reactions on a plurality of nucleic acid samples, base extension reactions are performed on the tag regions of the fifth nucleic acid sample and the sixth nucleic acid sample, and then base extension reactions are performed on the sequence regions of the fifth nucleic acid sample and the sixth nucleic acid sample;
optionally, the nucleic acid samples are fixed to a surface of a chip;
optionally, a plurality of liquid flow channels are arranged on the surface of the chip, and nucleic acid templates derived from different libraries are arranged in different channels, respectively.

9. The method according to any one of claims 1-8, wherein the sequencing system comprises a first sequencing system;
outputting sequencing data to an external server at nodes comprises:
S 100: in the case that base extension reactions of the first sequencing system are determined to have reached a first preset number of base extension cycles and a sequencing run is determined to have not yet been finished, connecting the first sequencing system and one or more specified external servers, starting transmission of at least part of the sequencing data to the external servers, and
starting monitoring of a transmission process,
the sequencing data comprising first sequencing data, which are signals generated by using the first sequencing system to perform base extension reaction runs until the first preset number of base extension cycles is reached; and
S500: in the case that transmission of the sequencing data is determined to have been finished, disconnecting the sequencing system and the external servers and ending the monitoring of the corresponding transmission process.

10. The method according to claim 9, further comprising, after S 100,
S200: when a second preset number of base extension cycles is determined to have been reached, starting the transmission of at least part of the sequencing data to the external servers,
the sequencing data comprising second sequencing data, which are signals generated by using the first sequencing system to perform base extension reaction runs until the second preset number of base extension cycles is reached from the first preset number of base extension cycles;
optionally, S200 further comprises:
in the case that transmission of the first sequencing data has not yet been finished, starting monitoring of a transmission process of the second sequencing data to the external servers.

11. The method according to claims 9 or 10, wherein the sequencing system further comprises a second sequencing system; the step of performing base extension reactions on nucleic acid samples further comprises: using the second sequencing system to perform the base extension reactions to generate sequencing data;
after S100, the method further comprises:
S300: in the case that the base extension reactions of the second sequencing system are determined to have reached a third preset number of base extension cycles and a sequencing run is determined to have not yet been finished, connecting the second sequencing system and one or more specified external servers, starting transmission of at least part of the sequencing data to the external servers, and starting monitoring of a transmission process,
the sequencing data comprising third sequencing data, which are signals generated by using the second sequencing system to perform base extension reaction runs until the third preset number of base extension cycles is reached.

12. The method according to any one of claims 9-11, wherein before starting the transmission of at least part of the sequencing data to the servers, S100 further comprises:
S102: configuring at least part of the sequencing data, including computing a first hash value that uniquely corresponds to the at least part of the sequencing data;
optionally, starting the transmission of at least part of the sequencing data to the servers comprises starting transmission of the first hash value to the servers, or S100 further comprises S104:
transmitting the first hash value to the servers;
optionally, further comprising, after S100,
S106: computing a second hash value that uniquely corresponds to the sequencing data transmitted to the servers, optionally, the first hash value and/or the second hash value being MDS value(s); and
S108: comparing the first hash value and the second hash value on the servers, determining that the transmission of the sequencing data has been finished if the first hash value and the second hash value are consistent, and determining that the transmission of the sequencing data has not yet been finished if the first hash value and the second hash value are inconsistent.

13. The method according to any one of claims 9-12, wherein the sequencing system comprises first data transmission software;
starting transmission of at least part of the sequencing data to the external servers comprises:
acquiring a sequencing intermediate data packet by the first data transmission software, the sequencing intermediate data packet comprising at least nucleic acid sample information derived from the sequencing systems and optical signal information generated by the base extension reactions; and
transmitting the sequencing intermediate data packet to the external servers by the first data transmission software through a communication network;
determining the base calling results by the external servers on the basis of the sequencing data comprises: completing base calling by the external servers on the basis of the sequencing intermediate data packet, and saving the obtained base calling results to a local folder;
optionally, the sequencing system further comprises sequencing software and a DC Server, and the external servers comprise second data transmission software that receives the sequencing intermediate data packet from the first data transmission software;
optionally, acquiring a sequencing intermediate data packet comprises:
acquiring the nucleic acid sample information from the sequencing software by the first data transmission software, and
acquiring the optical signal information generated by the base extension reactions from the DC Server by the first data transmission software.

14. The method according to any one of claims 9-13, further comprising, after S100,
S400: in the case that transmission of the sequencing data is determined to have not yet been finished and a connection between the sequencing system and the external servers is determined to be in an abnormal state, disconnecting the sequencing system and the external servers.

15. The method according to claim 14, wherein the abnormal state comprises at least one of the following cases:
(a) the transmission of the sequencing data is slower than a preset transmission speed;
(b) the transmission of the sequencing data is interrupted; and
(c) the external servers have insufficient operational space and/or physical space;
optionally, when the abnormal state comprises case (a), after disconnecting the sequencing system and the external servers, S400 further comprises:
restarting the transmission of the sequencing data to the external servers within a preset time and continuing the monitoring of the transmission process;
optionally, a criterion for deciding that the abnormal state comprises case (b) is that: within a preset time, an amount of data exchange is 0;
optionally, when the abnormal state comprises case (b), after disconnecting the sequencing system and the external servers, S400 further comprises:
active terminal network communication, restarting network communication after waiting for the preset time, restarting the transmission of the sequencing data to the external servers, and continuing the monitoring of the transmission process.
